# EUROPEAN PATENT SPECIFICATION

(11) **EP 1 960 104 B1**
(45) Date of publication and mention of the grant of the patent: **03.03.2010**
(21) Application number: 06820405.6
(22) Date of filing: 04.12.2006
(51) Int. Cl.: B01L 3/00, C12M 1/34, C12Q 1/68, G01K 11/12, G01N 33/18

(54) **APPARATUS FOR DETERMINING THE PRESENCE OF A CONTAMINANT IN A SAMPLE OF WATER OR OTHER FLUID**
VERFAHREN ZUR BESTIMMUNG DES VORLIEGENS EINER VERUNREINIGUNG IN EINER PROBE VON WASSER ODER EINES ANDEREN FLUIDS
APPAREIL DESTINE A DETERMINER LA PRESENCE D'UN CONTAMINANT DANS UN ECHANTILLON D'EAU OU D'UN AUTRE FLUIDE

(30) Priority: 03.12.2005 GB 0524770
(43) Date of publication of application: 27.08.2008
(73) Proprietor: The University of Bristol, Bristol BS8 1TH (GB)
(72) Inventor: GUNDRY, Stephen, Walter, Bath BA2 6BZ (GB)
(74) Representative: Wardle, Callum Tarn
(86) International application number: PCT/GB2006/004520
(87) International publication number: WO 2007/063337

(56) References cited:
- EP-A- 1 138 777
- WO-A1-01/23091
- WO-A1-99/59508
- US-A1- 4 235 964
- US-A1- 5 278 542
- US-A1- 2004 209 349
- US-A1- 2005 048 597

## Description

As the Millennium Development Goals for water recognise, microbially contaminated drinking water is a major cause of diarrhoeal disease, responsible for the deaths of 1.8 million people every year (WHO, 2004) most of which are children in developing countries. In contrast, the development of new water testing technologies is driven by the needs of water companies in North America and Europe to adhere to the stringent standards set by regulatory authorities and, more recently, to concerns about bio-terrorism. Even basic water testing equipment, skilled technicians and appropriate laboratory settings are rarely available in developing countries. As a result, there is a mismatch between the targets for technological development and the disease burden. This failure to develop appropriate diagnostics is analogous to the lack of investment by pharmaceutical companies to develop drugs to tackle diseases common only in developing countries.

When natural disasters occur, such as tsunami and earthquakes, agencies report that many of the attributable deaths are not the direct result of the disaster itself, but can be caused by subsequent outbreaks of disease, particularly from contaminated drinking water. Testing of drinking water sources after disasters presents particular problems due to the critical lack of staff, resources, and communications and transport infrastructure.

The World Health Organization issues Guidelines for Drinking-Water Quality. For bacteriological quality of drinking water, the WHO's webpage state *'(In) All water intended for drinking, E. coli or thermo tolerant coliform bacteria must not be detectable in any 100-ml sample'.* Whilst adherence to this stringent standard is required and achieved by most developed countries in the North, it is likely to be an unachievable target for most developing countries within the foreseeable future. This is particularly true where water is drawn from community sources in rural areas such as rivers or natural springs.

At present, many of the other available water testing technologies have been designed for use in developed countries. This is because the size of markets for water testing products is much greater in developed countries than in developing countries, where governments have only limited funds available for water testing. Many water testing technologies, such as the standard membrane filtration approach, require water samples to be collected in the field, stored under ice in transport containers, and then transported back to a microbiological laboratory. This microbiological laboratory needs to have appropriate facilities for testing samples, such as glassware incubators, lab benches, facilities for the disposal of potentially hazardous waste, refrigerators, and trained technicians capable of undertaking water tests.

In remote areas of developing countries, many of these facilities are simply unavailable. Ice for transporting water samples back to the laboratory may be impossible to obtain. The nearest microbiological laboratory may be a considerable distance away and there may be only very limited transport available for hard-pressed government environmental health technicians. Establishing a laboratory locally may also be difficult. Mains electricity may be either unavailable or available only sporadically and even buildings with workbenches and running water may be difficult to find. Many developing country organisations may be unable to afford the high consumables costs associated with some water tests. In many rural districts of developing countries, there is a lack of trained personnel able to carry out some of the more complex water testing procedures, such as calculating Most Probable Numbers of indicator bacteria or performing appropriate sample dilutions.

In recent years there has been some progress in the development of field kits for testing water. The University of Surrey developed the 'DelAgua' kit and this is still sold and used in the field, both in developing countries and by disaster relief agencies. It is based on the membrane filtration technique, requires a skilled technician and is time consuming. In more recent years, tests using Hydrogen Sulphide (H2S) have been developed to provide a simple 'Presence/Absence' result. An assessment of these tests (Sobsey and Pfaender, 2002) concluded (p37) *'The H2S method in various modifications has been tested in many places in different waters and produced results reported as indicating it to be a reasonable approach for testing treated and untreated waters for faecal contamination. It offers advantages including low cost (estimated at 20% of the cost of coliform assays), simplicity and ease of application to environmental samples.' However, the report noted several deficiencies in the reported assessments of the H2S test and commented 'Because of these deficiencies, it is not possible to widely and unequivocally recommend H2S tests for the determination of faecal contamination in drinking water. There remain too many uncertainties about the reliability, specificity and sensitivity of the test for detecting faecal contamination of drinking water and its sources.'*

Traditional laboratory tests include taking a 100ml sample of water and passing it through a filter membrane. The residue left on the filter membrane is then cultured with staining reagents. After a period of incubation, the stained colonies are counted manually. In recent years, several manufactures have produced reagents that use nutrient indicators to detect total coliforms and E.coli. Coliforms produce an enzyme that metabolises the nutrient indicators and cause either a change of colour or create fluorescence. These reagents are thus able to identify E.coli by visual or laser-based inspection. A known sample testing kit utilises one such nutrient indicator in conjunction with large sealable blister packs with has large numbers (50 - 97) of individual sample receiving wells. The nutrient indicator is mixed with a water sample which is then poured into the blister pack and the blister pack is subsequently sealed such that the individual wells are all filled with the sample and nutrient indicator mix. After an appropriate period of incubation the number of sample wells showing a positive result (indicative of contamination) is counted and statistical analysis applied to estimate the contamination level in cfu/100ml. However, the sample and nutrient mixing, the filling of the blister pack, the counting of the positive results and the statistical analysis all require skilled or educated personnel and as such are not suitable for use by untrained or uneducated individuals as is generally the case, for example, in developing countries.

US 2004/0 209 349 discloses an apparatus and method for testing liquid samples.

There is therefore a need for a method and apparatus for testing the quality of a fluid sample that substantially overcomes the above mentioned disadvantages.

According to a first aspect of the present invention there is provided apparatus for testing the quality of the fluid sample according to claim 1.

Preferably, the volume of at least one of the sample compartments differs from the volume of the other sample compartments. This allows an indication of the sample quality to be inferred simply from the number of sample compartments in which contamination is detected, since at low contamination levels only the sample compartments having the greater volumes will display contamination, whilst at greater contamination levels the smaller compartments will also display contamination.

Additionally or alternatively, the contaminant reagent retention means may comprise a rupturable membrane separating the plurality of contaminant reagent doses from respective sample compartments. Alternatively, the contaminant reagent retention means may comprise a permeable membrane located in each sample compartment, such that the contaminant reagent is permanently located within the sample compartment yet can mix with the water sample. In a further embodiment the contaminant reagent may be retained within a cartridge mechanism arranged such that the individual doses can be mechanically dispensed from the cartridge into the sample compartments, for example by means of linear or rotational movement of a dispensing member relative to the cartridge.

Additionally or alternatively, at least a portion of the sample compartments are transparent, or at least non-opaque, such that any visual indication provided by the contaminant reagent can be easily seen by the naked eye.

In preferred embodiments the apparatus may further comprise a first visual indicator arranged to indicate if the temperature of the apparatus has at any point fallen below a first threshold temperature value. Additionally, the apparatus may further comprise a second visual indicator arranged to indicate if the temperature of the apparatus has risen at any point above a second threshold temperature value. The lower and upper threshold values represent the extremes of temperature within which contaminant organisms have a significant growth rate (above the upper threshold the organisms are killed, whilst below the lower threshold their growth rate effectively stops). In preferred embodiments the visual indicators comprise a temperature sensitive chemical substance that undergoes a non-reversible change in appearance, such as colour, when a particular temperature threshold, be that upper or lower, is exceeded. The chemical substances may comprise temperature sensitive liquid crystals or leuco dyes.

In further preferred embodiments the apparatus may further include a third visual indicator arranged to indicate when the incubation period of the contaminant organism is complete. The third visual indicator may preferably be sensitive to the temperature of the apparatus, and thus the temperature of the samples being incubated. Additionally, the third visual indicator may preferably include a chemical substance that changes visual appearance, such as colour, at a rate equal to the growth rate of the contaminant. In other words, the third visual indicator mimics the temperature dependent behaviour of the contaminant. Suitable chemical substances include Time Temperature Indicators (TTIs) such as diffusion based indicators, enzymatic indicators or polymerisation reaction indicators.

Additionally or alternatively the apparatus may further comprise a heat source compartment arranged to receive a heat source, the heat source being provided to facilitate the incubation process. The apparatus may additionally comprise a heat source itself, such as a heated pad, one or more portions of exothermic chemicals, one or more portions of phase change materials, or any combination thereof. In the case of exothermic chemicals these may be encapsulated in a soluble material, preferably of varying thickness, such that the exothermic chemicals are triggered over a period of time as the encapsulating material dissolves. It is advantageous to provide one or more means of maintaining the temperature of the apparatus at a level suitable for good incubation of the contaminant organisms that does not rely on the availability of an external or 3^{rd} party power source, such as an electrical supply, since the apparatus may be used where no such power source is available.

Additionally or alternatively the apparatus may further comprise a neutralisation agent retention means arranged to retain a neutralising agent within the apparatus and arranged to dispense the neutralising agent into the sample compartments when actuated. The neutralisation retention means may comprise a rupturable membrane separating the neutralisation agent from the sample compartments. The purpose of the neutralisation agent is to both decontaminate the fluid sample after incubation, by killing any contaminating organisms, and to render the contaminant reagent itself harmless.

In a preferred embodiment the main body of the apparatus may be elongate and have first and second end faces, with the sample compartments comprising a plurality of elongate chambers extending between the end faces in the elongate body. This apparatus may further comprise at least one end cap arranged to be fastened over an end face and to seal the sample compartments in a fluid tight manner. The neutralising agent retention means may preferably be located within the end cap.

In an alternative embodiment the main body of the apparatus may comprise a planar element having a plurality of depressions, or wells, formed therein, the depressions constituting the sample compartments. The dose of contaminant reagent may be retained within each depression.

Embodiments of the present invention will be described below by way of non-limiting examples only, with reference to the accompanying figures of which:
Figure 1 shows an exploded view of a first embodiment of the present invention;
Figure 2 shows a detail view of an end cap of the apparatus of Figure 1;
Figure 3 shows a plan view of a second embodiment of the present invention;
Figure 4 shows a side view in cross-section of the embodiment of Figure 3; and
Figure 5 shows a side view of a further variant of the embodiment shown in Figures 3 and 4.

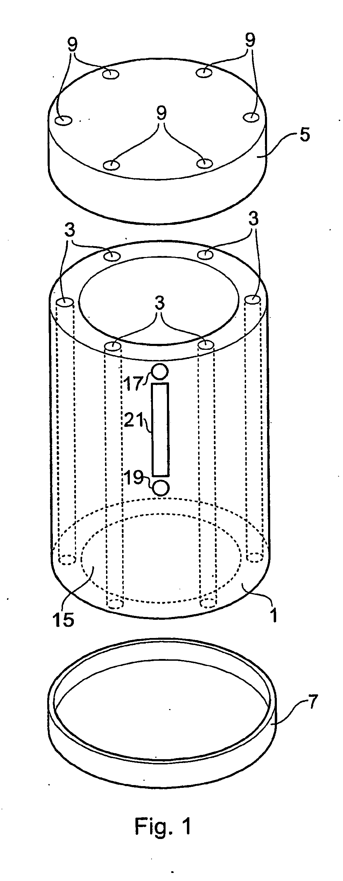

Figure 1 illustrates an exploded view of a first embodiment of the present invention. The water testing device comprises a main body 1 that is generally elongate in form and has a plurality of individual sample compartments 3 formed therein. In the embodiment illustrated the sample compartments comprise elongate passages extending through the full length of the main body 1 of the apparatus. In preferred embodiments ten separate sample compartments are provided (only a reduced number are illustrated in Figure 1 for the purposes of clarity). The main body 1 of the apparatus has first and second end faces. A first end cap 5 is provided that is arranged to fit over an end face of the main body 1 in a fluid tight manner, thus sealing one end of the sample compartments. A second end cap 7 is also provided that is similarly arranged to fit over the opposite end face of the main body 1 in a fluid tight manner, thus sealing the opposite end of the sample compartments. The first end cap 5 has a plurality of contaminant reagent doses 9 that are held within the end cap 5 by a contaminant reagent retention mechanism. The doses of contaminant reagent are located within the end cap 5 such that each dose is physically located adjacent to an end of a respective sample compartment when the end cap 5 is sealingly fastened over one end of the main body 1 of the apparatus. To ensure this spatial registration one or more cooperating engagement lugs may be provided on the end cap 5 and main body 1 of the apparatus (not illustrated in Figure 1). The contaminant reagent retention mechanism is arranged such that when desired the individual doses of contaminant reagent can be introduced into the corresponding sample compartments.

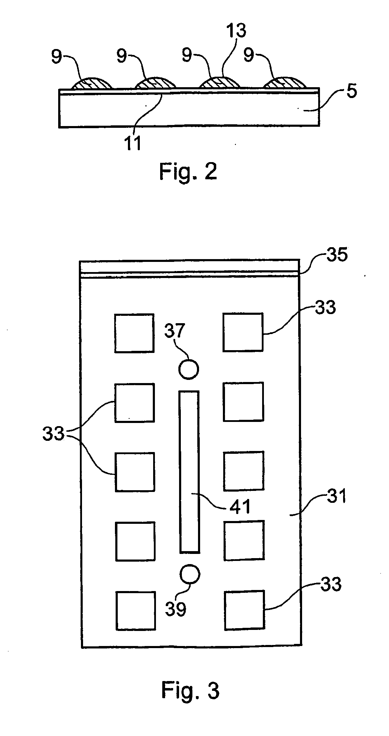

A first arrangement of the contaminant reagent mechanism is schematically illustrated in Figure 2. Figure 2 schematically illustrates a cross-sectional view of the first end cap 5 in which the contaminant reagent retention mechanism is located. The contaminant reagent retention mechanism comprises a rupturable membrane 11, such as a thin metal foil, that is bonded to the under surface of a blister pack 13 that itself is bonded to the external end face of the end cap 5. A number of blisters are formed in the blister pack, each blister containing a dose of the contaminant reagent. The blister pack is preferably manufactured from a deformable material, such as a deformable plastic, such that when a compressive force is applied above a certain threshold to the blister pack the contaminant reagent breaks the rupturable membrane 11 and is thus free to fall into the corresponding sample compartment. In an embodiment not forming part of the invention the contaminant reagent retention mechanism comprises separate mesh pockets located within each sample compartment, each mesh pocket containing a dose of the contaminant reagent, such that a fluid sample introduced into the sample compartment is free to mix with the contaminant reagent through the open pores of the mesh pocket. In a further embodiment of the invention the doses of contaminant reagent may be housed within a multiple compartment 'cartridge' that is arranged to be located within an appropriate recess within the end cap and an appropriate 'plunger' arrangement provided in the end cap that urges the individual doses from the cartridge, the plunger being mechanically linked to the cap such that linear or rotational movement of the cap causes the plunger to be urged towards the cartridge. Other mechanical retention and release mechanisms may be envisaged by those skilled within the art.

The opposite end cap 7 may include a neutralisation agent retention mechanism that may take a similar form to that of the contaminant reagent retention mechanism described above and which retains one or more doses of neutralisation agent that can be mixed with the contents of the sample compartments when required so as to render the contents of the sample compartment chemically and biologically inert. This is preferred since it allows the contents of the apparatus to be safely discarded after use without chemically or biologically contaminating the area in which disposal takes place.

In alternative embodiments only a single end cap may be provided, in which case one end of the main body 1 is formed without any openings. In this case both the contaminant reagent and neutralising agent retention mechanisms may be located within the single end cap.

To test a sample of a fluid, water for example, the individual sample compartments are filled with the water sample. This may most easily be accomplished by attaching one or the other of the end caps to the main body of the apparatus and immersing the apparatus in the source of water, if possible. Having filled the sample compartments both end caps are secured over the respective end faces of the main body of the apparatus so as to seal the individual sample compartments. The contaminant reagent retention mechanism is then actuated so as to introduce an individual dose of contaminant reagent into each of the sample compartments. The apparatus then needs to be incubated for a period of time to allow any contaminating organisms present in the sample to multiple to a detectable level. The range of temperatures over which any coliforms, for example, within the water sample will establish a colony is between 7°C and 44°C. Where this temperature cannot be maintained simply by virtue of the ambient temperature it is necessary to provide either an incubation heat source, insulation or cooling means. It is most probable that some form of heat source will be required rather than cooling. Because of the small size of the apparatus the necessary heating may be achieved by securing it against the human body (ideal for single test home use) or against the skin of a domestic animal. In the embodiment illustrated in Figure 1 the main body of the apparatus is substantially cylindrical with a central passage formed along the longitudinal axis of the main body and with the sample compartments located surrounding this central compartment. Thus the central compartment may be used to receive an appropriate heat source, for example a self contained pack of exothermic chemicals that are activated when the device is first immersed in the sample water source. However, it will be appreciated that other heat sources may be placed within the compartment, such as chemically heated elements triggered by the mixing of two or more exothermic chemicals, preheated thermal pads (preheated by immersion in heated water, for example) or solar or battery powered heating elements.

In some embodiments a portion of encapsulated exothermic chemicals are loaded into the central cavity, the encapsulation material being soluble such that on mixing with fluid (taken from the sample fluid) the exothermic chemicals are activated only after the encapsulation has been dissolved, thus providing a time delay in triggering the heating action. By varying the thickness of encapsulation the rate at which the exothermic chemicals are triggered can be controlled so as to prolong the overall heating effect. In some embodiments one or more of the provided end caps may contain the encapsulated exothermic chemicals such that they may be released into the central cavity of the apparatus when required.

In other embodiments the heat source may be provided by the inclusion of phase change materials within the apparatus, which are characterised by the property of either extracting heat from or imparting heat to any surrounding material as they change phase, for example from the solid to the liquid phase. Thus the cavity may be filled with a phase change material that imparts heat to its surroundings as it changes from the liquid to the solid phase and this may be triggered simply by placing the filled apparatus in a direct heat source, such as in the direct sunlight. Equally, the walls of the apparatus may be formed so as to enclosed one or more pockets of such phase change material so as to replace or augment the use of the central cavity.

The ability to heat or cool the apparatus without an external power supply (or with only a limited power supply) is particularly advantageous in circumstances where the apparatus is used in very rural or remote locations where a permanent or reliable power supply may not be available.

In ideal laboratory conditions the fluid samples may be incubated at 35°C for a period of approximately 18 hours, for example. However, due to the intended use in non laboratory conditions it cannot be guaranteed that such a constant temperature will be maintained and therefore the period of incubation will vary as a function of the temperature profile to which the device is exposed during incubation. Consequently, in preferred embodiments of the present invention one or more visual indicators are provided to indicate in an unambiguous manner whether or not incubation has been completed and whether or not it has been successful. In terms of the success of the incubation when the contaminant of interest is E.coli or other coliforms, incubation will not be successful if the temperature of the device is allowed to fall below the previously mentioned minimum temperature of approximately 7°C or above the upper threshold temperature value of approximately 44°C. Consequently, a first visual indicator 17 may be provided that preferably comprises an appropriate temperature sensitive chemical substance that if exposed to a temperature above approximately 44°C will undergo a non-reversible change in appearance. Most preferably, the chemical substance is selected such that on exposure to temperature above the threshold value it will change colour to a red colour, thus visually indicating that the device has been exposed to an excessive temperature and that incubation will not be valid. A second visual indicator 19 may also be provided, again preferably comprising an appropriate chemical substance, that when exposed to a temperature below the lower threshold value of approximately 7°C undergoes an non-reversible change in appearance, preferably changing appearance to a blue colour and thus indicating that the device has been exposed to a temperature below the accepted minimum and thus that the incubation will have prematurely ceased. Examples of suitable chemicals include liquid crystals or leuco dyes. Liquid crystals use organic polymers like cholestryl nonanoate or cyanobiphenyls that change their orientation with temperature such that the relative change in crystal shapes is in the visible light spectrum, thus resulting in a colour change when viewed by the human eye. Alternatively they cut visible light out completely and go coloured to black. These liquid crystals are encapsulated and suspended in a paint medium. Other transparent to coloured organic polymers (i.e. leuco dyes) are spirolactanes, fluorans, spiropyrans and fulgides. In the embodiment illustrated in Figure 1 the first and second visual indicators take the form of small disks or circles located on the outer surface of the main body 1 of the device, although they may be located elsewhere on the apparatus.

Also located on the outer surface of the main body is a third visual indicator that is arranged to indicate when the incubation process has been successfully completed. As previously mentioned, the time period required for successful incubation, assuming appropriate temperature conditions, will nonetheless vary depending upon the range of temperatures to which the device has been exposed. Consequently, in preferred embodiments the third visual indicator 21 comprises a chemical substance that is arranged to change visual appearance over a period of time and such that the rate at which the substance changes appearance closely matches the rate of incubation of the coliforms depending on the exposed temperature curve. In other words, the rate at which the chemical substance changes visual appearance is dependent upon the temperature to which it is exposed to. Suitable chemical substances are Time-Temperature-Indicators/Integrators (TTIs). These fall into 3 main types: diffusion based indicators, enzymatic indicators, and solid state polymerization reaction indicators. The latter group are compounds that undergo addition polymerisation to give a progressive irreversible colour change that is indicative of the integrated time - temperature conditions. In preferred embodiments, and as illustrated in Figure 1, the third visual indicator comprises a rectangular strip that changes visual appearance in a progressive manner over the incubation period such that when the entirety of the strip has changed visual appearance then incubation is deemed to have been completed.

It will of course be appreciated by those skilled in the art that other non-chemical visual indicators may be provided that have the same functionality as the chemical substances described above in relation to the first, second and third visual indicators. For example, an electronic indication mechanism may easily be conceived utilising one or more temperature sensors, appropriate thresholding circuitry and visual displays. However, whilst possible and within the scope of the current invention, these non-chemical solutions are not preferred because of their increased complexity and cost.

In further embodiments, rather than providing a heat source, or cooling source, within the central cavity 15 of the device, a plastic sleeve may be provided that is arranged to fit over the outside of the apparatus as illustrated in Figure 1, the plastic sleeve optionally including either pockets for separate heat sources or cooling means or itself including an exothermic heat source. In further embodiments an electrically activated heating or cooling mechanism may be provided that is either battery powered or solar powered, in conjunction with a provided solar panel.

As previously discussed, regulatory authorities have accepted the membrane filtration and Most Probable Number methods to establish a quantified estimate of the contamination level in terms of cfu/100ml. Apart from being overly complicated to determine in the context of non-laboratory conditions and unskilled users, these methodologies provide a degree of quantification that is more than that required in the context of simply providing an indication of the general quality level of the sampled water source. However, it is still desirable to provide some indication of differing quality levels beyond merely an indication of the presence or absence of faecal contamination. This is desirable where it is probable that children or immuneo-compromised individuals will be the recipients of the water, in which case it is preferable for the water given to these individuals to be of a higher quality than might otherwise be acceptable.

With embodiments of the present invention the quality of the water sample may be differentiated between three different levels of contamination, for example 0 to 10 cfu/100ml, 10 to 100 cfu/100ml and 100 + cfu/100ml. The applicant has determined that the minimum sample required to distinguish, with a 95% confidence level, between contamination of less than 10 cfu/100ml or more than 10 cfu/100ml is 37.5ml. The applicant has also realised that at higher levels of contamination only a smaller sample size is required for the contamination reagent to provide a visual indication of contamination. Consequently, in preferred embodiments of the present invention ten sample compartments are provided of differing volumes, for example 2x10ml, 2x5ml, 2x2.5ml, 2x1ml and 2x0.5ml, a total volume of 38ml i.e. above the minimum required to distinguish the lowest contamination level at the 95% confidence level. By virtue of the different volumes of the sample compartments it is possible by simply counting the number of compartments that demonstrate visual indication of contamination to determine the likely overall level of contamination. Furthermore, it is not necessary to distinguish between the differing sizes of compartments that demonstrate the visual indication, merely the overall number of compartments. Consequently, a simple chart may be provided with the apparatus correlating the number of compartments that show a visual indication of contamination with the approximate contamination level. Ten compartments are provided in preferred embodiments of the apparatus since even innumerate individuals are likely to be able to count up to 10. Equally, by taking into account which of the sample compartments show contamination a more precise indication of the level of contamination may be inferred by more skilled users. It will also be appreciated that the number and the overall volume of the sample compartments may be varied if confidence levels other than 95% are either required or are acceptable. For example, for a greater level of confidence the overall volume of the sample compartments must be increased.

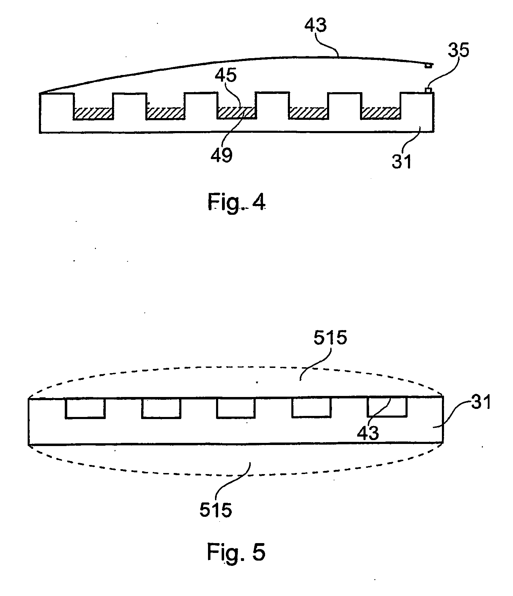

An alternative embodiment of the apparatus of the present invention is schematically illustrated in Figures 3 to 5. A plan view is shown in Figure 3 in which a main planar element 31 is provided having a plurality of sample compartments 33 formed therein. A seal 35 is provided for closing the apparatus after the water sample has been introduced into it. First, second and third visual indicators 37, 39 and 41 are provided on the main element and may be implemented as previously discussed with respect to the embodiment illustrated in Figure 1. Figure 4 shows a side view of the apparatus of Figure 3 taken through a cross section through one set of the sample compartments 33. The sample compartments are formed as depressions or wells within the main element 31. A sealable cover 43 is provided that is preferably permanently attached at one end of the main element 31 and has one part of the seal 35 at the opposite end thereof. In use, the seal 35 is opened, thus allowing the sample fluid to be introduced into the apparatus. The sealable cover is then closed over the main element 31 and sealed thereto by means of the seal 35, the cover 43 thus isolating the individual sample compartments 33. In the embodiment illustrated in Figure 4, a permeable membrane 45 is provided part way down the sidewall of the depressions forming the sample compartments, the permeable membrane being provided to restrain individual doses of contaminant reagent 49 within the individual sample compartments. Figure 5 illustrates a further variation of the embodiment illustrated in Figures 3 and 4, in which an additional compartment 5 on 5 may be provided, for example by means of a further flexible membrane, so as to receive a heating source to aid incubation as previously discussed.

In preferred embodiments the contaminant reagent comprises a nutrient indicator that produces a visible colour change to indicate the presence of a contaminant after the incubation period. Therefore in preferred embodiments at least a portion of each of the sample compartment is transparent or non-opaque to allow visual inspection of the contents to be made. Where the sample compartments are of differing volumes it is also preferable for the non-opaque part of the sample compartments to all be of the same size and appearance so that it is not readily apparent which compartment is which, since this may affect how the test results are reported by untrained users. Whilst this may be achieved by simply varying the size of the sample compartments behind the transparent 'windows', this may have the effect of varying the depth of perceived colour between separate compartments, due to the possible variation in thickness of fluid sample being viewed. Since this may itself be undesirable (as an untrained or experienced user may falsely discount a sample compartment apparently only showing a light shade of contaminant indicator as uncontaminated), it is more preferable for the internal geometry of the sample compartments to be such that the physical depth of the compartment opposite the transparent portion is the same regardless of the overall volume of the compartment.

Although primarily intended for use in rural or remote areas of the world where full laboratory facilities are not available, it will be appreciated that the apparatus may be used in other situations, such as in military applications or in disaster areas. It will also be appreciated that although the embodiments described above have mostly referred to drinking water quality the apparatus of the present application may be used for other water sources, such as river or lake water, or even other fluids, such as animal milk.

## Claims

1. Apparatus for testing the quality of a fluid sample, the apparatus comprising:
a main body including a plurality of sample compartments,
a contaminant reagent retention means arranged to retain a plurality of doses of contaminant reagent within the apparatus and arranged to allow a dose of contaminant reagent to be added to a fluid sample in a respective one of the sample compartments, and
a first cap for closing fluid under test within one or more of said compartments,
**characterised in that** said retention means is located within said first cap.

2. Apparatus according to claim 1, wherein the volume of at least one of the sample compartments differs from the volume of the other sample compartments.

3. Apparatus according to any preceding claim, wherein the contaminant reagent retention means comprises a rupturable membrane separating the plurality of reagent doses from respective sample compartments.

4. Apparatus according to any preceding claim, wherein said sample compartments are non-opaque.

5. Apparatus according to any preceding claim further comprising a first visual indicator arranged to indicate if the temperature of the apparatus had fallen below a first threshold temperature value.

6. Apparatus according to any preceding claim further comprising a second visual indicator arranged to indicate if the temperature of the apparatus had risen above a second threshold temperature value.

7. Apparatus according to claim 5 or 6, wherein said visual indicator comprises a temperature sensitive chemical substance that undergoes a non-reversible change in appearance when a temperature threshold is exceeded.

8. Apparatus according to any preceding claim further comprising a third visual indicator arranged to indicate when the incubation period of the contaminant reagent is complete.

9. Apparatus according to claim 8, wherein the third visual indicator is sensitive to the temperature of the apparatus.

10. Apparatus according to claim 9, wherein the third visual indicator includes a chemical substance that changes visual appearance at a rate equal to that of the contaminant reagent for the experienced temperature of the apparatus.

11. Apparatus according to any preceding claim further comprising a heat source compartment arranged to receive a heat source.

12. Apparatus according to any preceding claim further comprising a heat source.

13. Apparatus according to any preceding claim further comprising a contaminant reagent neutralisation retention means arranged to retain a neutralising agent within the apparatus and arranged to dispense the neutralising agent into the sample compartments when actuated.

14. Apparatus according to claim 13, wherein the neutralisation retention means comprises a rupturable membrane separating the neutralisation agent from the sample compartments.

15. Apparatus according to any preceding claim, wherein said main body is elongate and has first and second end faces and wherein the sample compartments comprise a plurality of elongate chambers extending between said end faces.

16. Apparatus according to claim 15, wherein the apparatus further comprising a second cap and wherein said caps are arranged to be fastened over the respective end faces and seal said sample compartments in a fluid tight manner.

17. Apparatus according to any preceding claim, wherein said main body comprises a planar element having a plurality of depressions formed therein, said depressions constituting said sample compartments.

## Patentansprüche

1. Vorrichtung zum Testen der Qualität einer Fluidprobe, wobei die Vorrichtung aufweist:
einen Hauptkörper mit mehreren Probenkammern;
eine Kontaminantreagenzaufnahmeeinrichtung, die dazu geeignet ist, mehrere Kontaminantreagenzdosen in der Vorrichtung aufzunehmen, und dazu geeignet ist, einer Fluidprobe in einer entsprechenden der Probenkammern eine Kontaminantreagenzdosis zuzuführen; und
eine erste Kappe zum Verschließen von zu testendem Fluid in einer oder in mehreren der Probenkammern;
**dadurch gekennzeichnet, dass**
die Kontaminantreagenzaufnahmeeinrichtung in der ersten Kappe angeordnet ist.

2. Vorrichtung nach Anspruch 1, wobei das Volumen mindestens einer der Probenkammern sich vom Volumen der anderen Probenkammern unterscheidet.

3. Vorrichtung nach Anspruch 1 oder 2, wobei die Kontaminantreagenzaufnahmeeinrichtung eine reißfähige Membran aufweist, die die mehreren Reagenzdosen von den jeweiligen Probenkammern trennt.

4. Vorrichtung nach einem der vorangehenden Ansprüche, wobei die Probenkammern nicht-opak sind.

5. Vorrichtung nach einem der vorangehenden Ansprüche, ferner mit einer ersten optischen Anzeige, die dazu geeignet ist, anzuzeigen, wenn die Temperatur der Vorrichtung unter einen ersten Schwellentemperaturwert abgesunken ist.

6. Vorrichtung nach einem der vorangehenden Ansprüche, ferner mit einer zweiten optischen Anzeige, die dazu geeignet ist, anzuzeigen, wenn die Temperatur der Vorrichtung über einen zweiten Schwellentemperaturwert angestiegen ist.

7. Vorrichtung nach Anspruch 5 oder 6, wobei die optische Anzeige eine temperaturempfindliche chemische Substanz aufweist, die eine nichtreversible Änderung ihres Erscheinungsbildes erfährt, wenn ein Temperaturschwellenwert unter- bzw. überschritten wird.

8. Vorrichtung nach einem der vorangehenden Ansprüche, ferner mit einer dritten optischen Anzeige, die dazu geeignet ist, anzuzeigen, wenn die Inkubationsperiode des Kontaminantreagenz abgeschlossen ist.

9. Vorrichtung nach Anspruch 8, wobei die dritte optische Anzeige auf die Temperatur der Vorrichtung anspricht.

10. Vorrichtung nach Anspruch 9, wobei die dritte optische Anzeige eine chemische Substanz aufweist, deren optisches Erscheinungsbild sich mit einer Rate ändert, die derjenigen des Kontaminantreagenz für die jeweilige Temperatur der Vorrichtung gleicht.

11. Vorrichtung nach einem der vorangehenden Ansprüche, ferner mit einer Wärmequellenkammer, die dazu geeignet ist, eine Wärmequelle aufzunehmen.

12. Vorrichtung nach einem der vorangehenden Ansprüche, ferner mit einer Wärmequelle.

13. Vorrichtung nach einem der vorangehenden Ansprüche, ferner mit einer Kontaminantreagenzneutralisationsmittelaufnahmeeinricht ung, die dazu geeignet ist, ein Neutralisationsmittel in der Vorrichtung aufzunehmen, und dazu geeignet ist, das Neutralisationsmittel in die Probenkammern abzugeben, wenn sie aktiviert wird.

14. Vorrichtung nach Anspruch 13, wobei die Neutralisationsmittelaufnahmeeinrichtung eine reißfähige Membran aufweist, die das Neutralisationsmittel von den Probenkammern trennt.

15. Vorrichtung nach einem der vorangehenden Ansprüche, wobei der Hauptkörper länglich ist und eine erste und eine zweite Endfläche aufweist, und wobei die Probenkammern mehrere sich zwischen den Endflächen erstreckende längliche Hohlräume sind.

16. Vorrichtung nach Anspruch 15, wobei die Vorrichtung ferner eine zweite Kappe aufweist, und wobei die Kappen dazu geeignet sind, über den entsprechenden Endflächen befestigt zu werden und die Probenkammern auf eine fluiddichte Weise abzudichten.

17. Vorrichtung nach einem der vorangehenden Ansprüche, wobei der Hauptkörper ein flaches Element mit mehreren darin ausgebildeten Vertiefungen ist, wobei die Vertiefungen die Probenkammern bilden.

## Revendications

1. Appareil pour évaluer la qualité d'un échantillon de fluide, l'appareil comprenant :
un corps principal incluant plusieurs compartiments d'échantillon,
un moyen de rétention de réactif de contaminant agencé pour retenir plusieurs doses de réactif de contaminant à l'intérieur de l'appareil et agencé pour permettre d'ajouter une dose de réactif de contaminant à un échantillon de fluide dans un compartiment respectif des compartiments d'échantillon, et
un premier capuchon pour enfermer un fluide en cours d'évaluation à l'intérieur d'un ou de plusieurs desdits compartiments,
**caractérisé en ce que** ledit moyen de rétention est situé à l'intérieur dudit premier capuchon.

2. Appareil selon la revendication 1, dans lequel le volume d'au moins un des compartiments d'échantillon diffère du volume des autres compartiments d'échantillon.

3. Appareil selon l'une quelconque des revendications précédentes, dans lequel le moyen de rétention de réactif de contaminant comprend une membrane pouvant être rompue séparant les plusieurs doses de réactif des compartiments d'échantillon respectifs.

4. Appareil selon l'une quelconque des revendications précédentes, dans lequel lesdits compartiments d'échantillonnage sont non opaques.

5. Appareil selon l'une quelconque des revendications précédentes comprenant en outre un premier indicateur visuel agencé pour indiquer si la température de l'appareil est tombée au-dessous d'une première valeur de température de seuil.

6. Appareil selon l'une quelconque des revendications précédentes comprenant en outre un deuxième indicateur visuel agencé pour indiquer si la température de l'appareil a dépassé une seconde valeur de température de seuil.

7. Appareil selon la revendication 5 ou 6, dans lequel ledit indicateur visuel comprend une substance chimique sensible à la température qui subit un changement d'apparence non réversible quand un seuil de température est dépassé.

8. Appareil selon l'une quelconque des revendications précédentes, comprenant en outre un troisième indicateur visuel agencé pour indiquer quand la période d'incubation du réactif de contaminant est achevée.

9. Appareil selon la revendication 8, dans lequel le troisième indicateur visuel est sensible à la température de l'appareil.

10. Appareil selon la revendication 9, dans lequel le troisième indicateur visuel comprend une substance chimique qui change d'apparence visuelle à une vitesse égale à celle du réactif de contaminant pour la température subie de l'appareil.

11. Appareil selon l'une quelconque des revendications précédentes, comprenant en outre un compartiment de source de chaleur agencé pour recevoir une source de chaleur.

12. Appareil selon l'une quelconque des revendications précédentes, comprenant en outre une source de chaleur.

13. Appareil selon l'une quelconque des revendications précédentes, comprenant en outre un moyen de rétention de neutralisation de réactif de contaminant agencé pour retenir un agent de neutralisation à l'intérieur de l'appareil et agencé pour distribuer l'agent de neutralisation dans les compartiments d'échantillon lors de la mise en oeuvre.

14. Appareil selon la revendication 13, dans lequel le moyen de rétention de neutralisation comprend une membrane pouvant être rompue séparant l'agent de neutralisation des compartiments d'échantillon.

15. Appareil selon l'une quelconque des revendications précédentes, dans lequel ledit corps principal est allongé et a des première et seconde faces d'extrémité et dans lequel les compartiments d'échantillon comprennent plusieurs chambres allongées s'étendant entre lesdites faces d'extrémité.

16. Appareil selon la revendication 15, dans lequel l'appareil comprend en outre un second capuchon et dans lequel lesdits capuchons sont agencés pour être attachés au-dessus des faces d'extrémité respectives et scellent lesdits compartiments d'échantillon d'une façon étanche au fluide.

17. Appareil selon l'une quelconque des revendications précédentes, dans lequel ledit corps principal comprend un élément plan ayant plusieurs dépressions formées en son sein, lesdites dépressions constituant lesdits compartiments d'échantillon.
